# EUROPÄISCHE PATENTSCHRIFT

(11) **EP 0 374 651 B1**
(45) Veröffentlichungstag und Bekanntmachung des Hinweises auf die Patenterteilung: **29.09.1993**
(21) Anmeldenummer: 89122758.9
(22) Anmeldetag: 09.12.1989
(51) Int. Cl.: C12P 41/00, C12P 13/04, C12N 9/10, C12P 9/00

(54) **Verfahren zur Herstellung von L-Phosphinothricin**
Process for the preparation of L-phosphinothricine
Procédé de préparation de L-phosphinothricine

(30) Priorität: 14.12.1988 DE 3842025
(43) Veröffentlichungstag der Anmeldung: 27.06.1990
(73) Patentinhaber: HOECHST AKTIENGESELLSCHAFT, 65926 Frankfurt am Main (DE)
(72) Erfinder: Then, Johann, Dr., D-6230 Frankfurt am Main 80 (DE); Aretz, Werner, Dr., D-6240 Königstein/Taunus (DE); Sauber, Klaus, Dr., D-6232 Bad Soden am Taunus (DE)

(56) Entgegenhaltungen:
- EP-A- 0 248 357
- EP-A- 0 249 188
- FR-A- 2 438 054
- CHEMICAL ABSTRACTS, Band 69, Nr. 15, 7. October 1968, Seite 5447, Zusammenfassung Nr. 58390t, Columbus, Ohio, US

## Beschreibung

L-2-Amino-4-methylphosphinobuttersäure (im folgenden als L-Phosphinothricin oder L-PTC bezeichnet) oder deren Salze sind - wie auch aus DE-OS 29 39 269 bekannt geworden ist - die wirksame Komponente der chemisch leicht zugänglichen Racemate. Letztere besitzen gemäß DE-OS 27 17 440 eine sehr gute und breite herbizide Wirksamkeit gegenüber zahlreichen monokotylen und dikotylen, einjährigen und mehrjährigen Unkräutern. Da L-PTC und seine oben angeführten Derivate im Vergleich zu den Racematen etwa doppelt so stark wirksam sind, ist es wünschenswert, ein Verfahren zu entwickeln, mit dem es möglich ist, L-PTC auf einfache Weise in größeren Mengen zugänglich zu machen.

Mit Hilfe einer D-Aminosäure-Transaminase und einer L-Aminosäure-Transaminase kann auf elegante Weise die Racematspaltung von D,L-Phosphinothricin sowie die Überführung von D-PTC in L-PTC durchgeführt werden.

Die Erfindung betrifft somit ein Verfahren zur Herstellung von L-2-Amino-4-methylphosphinobuttersäure, das dadurch gekennzeichnet ist, daß D,L-2-Amino-4-methylphosphinobuttersäure in Gegenwart von D-Aminosäure- und L-Aminosäure-Transaminase mit α-Ketosäuren und Aminogruppendonatoren umgesetzt wird.

Die Erfindung wird im folgenden insbesondere in ihren bevorzugten Ausführungsformen detailliert erläutert. Ferner wird die Erfindung in den Ansprüchen definiert.

Die D-Aminosäure-Transaminase kann aus Pseudomonas putida und Bacillus licheniformis gewonnen werden (Deutsche Offenlegungsschrift 34 47 023 und Niederländische Offenlegungsschrift 87 02 449). Bevorzugt wird die in der Deutschen Offenlegungsschrift DE 34 47 023 beschriebene D-Transaminase eingesetzt, die aus Bacillus licheniformis ATCC 9945 isoliert wird.

Als L-Aminosäure-Transaminase können entsprechende Enzyme aus E. coli, Paracoccus denitrificans, Torula oder Rhodotorula (Deutsche Offenlegungsschrift 34 23 936) Anwendung finden. Bevorzugt wird hier die L-Transaminase aus E. coli ATCC 11303 verwendet, die beispielsweise auch in J. Then et al., Biotechnology Letters 9 (10), 680 - 684 (1987) beschrieben wurde.

Die erfindungsgemäße Racematspaltung gliedert sich in zwei Reaktionsschritte
1. D-Aminosäure-Transaminierung:
   D,L-Phosphinothricin +
   α-Ketosäure, in Gegenwart von
   D-Aminosäure-Transaminase ergibt
   D-Aminosäure + 3-Carboxy-3-oxo-propylmethyl-phosphinat +
   L-Phosphinothricin
   and anschließend

2. L-Aminosäure-Transaminierung:
   3-Carboxy-3-oxo-propylmethyl-phosphinat +
   L-Aminosäure, in Gegenwart von
   L-Aminosäure-Transaminase ergibt
   L-Phosphinothricin +
   α-Ketosäure.

Zur Durchführung der erfindungemäßen Reaktion können jeweils die nach an sich bekannten Verfahren isolierten Enzyme oder auch die ganzen Zellen in freier oder immobilisierter Form eingesetzt werden.

Als Aminogruppendonator für den ersten Reaktionsschritt wird bevorzugt D,L-PTC und für den zweiten Reaktionsschritt L-Asparagin, Glycin, L-Asparaginsäure und L-Glutaminsäure eingesetzt. Die Aminosäuren werden in Form ihrer freien Säuren oder geeigneten Salze verwendet.

Als α-Ketosäure im ersten Reaktionsschritt kommen vorzugsweise Phenylglyoxylat, 4-Hydroxyphenylglyoxylat und Thiophenylglyoxylat zur Anwendung.

Die Menge der D-Aminosäure-Transaminase im Reaktionsansatz kann in einem weiten Bereich schwanken. Zweckmäßig liegt sie in einem Bereich von 0,05 bis 50 µmol/min·ml. Vorzugsweise enthält der Ansatz 0,1 bis 2 µmol/min·ml.

Das racemische Gemisch von PTC wird in Bezug auf die α-Ketosäure zweckmäßig in doppelter molarer Menge eingesetzt. Um möglicherweise das Gleichgewicht zum Endprodukt der Reaktion zu verschieben, wird die α-Ketosäure in geringfügig höheren Mengen eingesetzt.

Die Zugabe der Reaktionskomponenten zum Reaktionsansatz kann als Lösung in Wasser oder als feste Substanzen gleichzeitig erfolgen. Bevorzugt ist jedoch eine gestaffelte Zugabe in Mengen von 1 bis 5 %, insbesondere 1,5 bis 4,5 %, jeweils bezogen auf das Gemisch des Reaktionsansatzes, über einen Zeitraum von 1 bis 90 Stunden. Es wird vorteilhaft bei einem pH-Wert zwischen 5 und 9, insbesondere zwischen 7 und 8,5, gearbeitet. Weiterhin ist es zweckmäßig, die Reaktion in einem Temperaturbereich von 20 bis 65°C durchzuführen.

Die Enzymmenge der L-Aminosäure-Transaminase in den Reaktionsansätzen kann aus einem weiten Bereich gewählt werden. Zweckmäßig liegt sie zwischen 10 bis 20 000 µmol/min·l. Vorzugsweise enthält der Ansatz Enzymmengen von 1 500 bis 20 000 µmol/min·l.

Der Aminogruppendonator für die L-Aminosäure-Transaminase wird in äquimolaren Mengen bzw. im Überschuß zur α-Ketosäure eingesetzt, die im ersten Reaktionsschritt gebildet wird. Verhältnisse von 1:1 bis 5:1, vorteilhaft 1:1 bis 2:1, haben sich bewährt. Die Zugabe des Aminogruppendonators erfolgt entweder sofort oder gestaffelt über die gesamte Reaktionszeit. Es ist vorteilhaft bei einem pH-Wert zwischen 5 und 9, inbesondere zwischen 7 und 8,5 zu arbeiten. Es ist auch vorteilhaft die Reaktion in einem Temperaturbereich von 20 bis 65°C durchzuführen.

Insbesondere bevorzugt wird das Verfahren als sogenanntes "Eintopf-Verfahren" durchgeführt. Dabei werden die Enzyme L-Aminosäure-Transaminase und D-Aminosäure-Transaminase zusammen in Lösung oder an einen Träger gebunden, wie in DOS 32 37 341 oder in DOS 33 44 912 beschrieben, verwendet. Ebenso werden die Reaktionskomponenten zusammen in einen Ansatz gegeben. Dieses "Eintopf-Verfahren" kann insbesondere deshalb so vorteilhaft angewendet werden, weil die L-Aminosäure-Transaminase nicht mit den α-Ketosäuren des ersten Reaktionsschritts, Phenylglyoxylat, 4-Hydroxyphenylglyoxylat und Thiophenylglyoxylat als Substrat reagiert. Die D-Aminosäure-Transaminase, auf der anderen Seite, reagiert nicht mit den L-Aminosäuren des zweiten Reaktionsschritts. Ferner reagieren die Enzyme im gleichen pH- und Temperaturbereich.

Die erfindungsgemäße Racematspaltung hat folgende Vorteile gegenüber den herkömmlichen Verfahren zur Racematspaltung von D,L-Phosphinothricin:
- keine Salzbelastung infolge Einstellung stark unterschiedlicher pH-Werte, wie z.B. bei der Racematspaltung mit Penicillin-G-acylase
- Keine Verluste wie bei der thermischen Racemisierung.
- Es kann nicht nur L-PTC als Wertprodukt hergestellt werden, sondern auch zusätzlich noch eine andere D-Aminosäure.

### Beispiel 1

Mit D-Aminosäure-Transaminase aus Bacillus licheniformis ATCC 9945 (DOS 34 47 023, Beispiele 1 bis 4, siehe Anhang) wurde folgender Reaktionsansatz inkubiert:

| | | |
|---|---|---|
| D,L-Phosphinothricin | (200 mM) | 0,2 ml |
| α-Ketosäure | (100 mM) | 0,2 ml |
| K-Phosphat-Puffer | ( 50 mM; pH 8,0) | 0,3 ml |
| Pyridoxalphosphat | (200 µg/ml) | 0,05 ml |
| D-Aminosäure-Transaminase-Rohextrakt (aus Beispiel 3, Anhang) | | 0,25 ml |

Die Ansätze wurden 24 Stunden bei 37°C mit 180 UpM geschüttelt. Die folgende Tabelle zeigt die Ergebnisse:

| Aminogruppendonator | Aminogruppenakzeptor | Umsetzung |
|---|---|---|
| D,L-Phosphinothricin | Pyruvat | + |
| D,L-Phosphinothricin | Phenylpyruvat | ++ |
| D,L-Phosphinothricin | 4-Hydroxyphenylglyoxylat | ++ |
| Je 1 µl der obigen Ansätze wurde auf Kieselgel-Dünnschichtplatten aufgetragen und im Laufmittel Butanol-Eisessig-Wasser (4:1:1) aufgetrennt. Die Identifizierung erfolgte durch Referenzsubstanzen nach Besprühen mit Ninhydrin. ++ gute Umsetzung + deutliche Umsetzung | | |

Zur Messung der D-Aminosäure-Transaminase-Aktivität werden D-α-Aminoadipinsäure (20 mmolar), a-Ketoglutarat (10 mmolar) und Pyridoxalphosphat (10 µg/ml) in Kaliumphosphatpuffer (10 mmolar), pH 8, bei einer Temperatur von 37°C mit dem Enzym inkubiert. Dabei entsteht D-Glutaminsäure, die dünnschichtchromatographisch nach Besprühung mit Ninhydrin bestimmt werden kann.

### Beispiel 2

### Herstellung der L-Aminosäure-Transaminase

E. coli ATCC 11303 wurde in folgender Nährlösung angezogen.

| | |
|---|---|
| Fumarsäure | 5 g/l |
| Fleischextrakt | 20 g/l |
| Asparaginsäure | 20 g/l |
| KH₂PO₄ | 2 g/l |
| MgSO₄·7H₂O | 0,5 g/l |
| CaCl₂·2H₂O | 0,1 g/l; pH 7,2 |

Nach 20 Stunden Schütteln bei 200 UpM und 37°C wurden die Zellen in K-Phosphatpuffer (50 mM, pH 7,4) suspendiert und abzentrifugiert. Die Zellen wurden anschließend erneut mit 1 ml Phosphatpuffer pro g Zellen aufgenommen und durch Zugabe von 27 µmol/l N-Cetyl-N,N,N-trimethylammoniumbromid aufgeschlossen. Anschließend wurde die Suspension erneut zentrifugiert. Im Überstand befindet sich die L-Aminosäure-Transaminase.

### Beispiel 3

Es wurde, wie in Beispiel 1, ein Reaktionsansatz von D,L-Phosphinothricin und 4-Hydroxyphenylglyoxylat von 5 ml Gesamtvolumen 24 h mit D-Aminosäure-Transaminase inkubiert. Dann wurden 0,02 g L-Asparaginsäure-Na-Salz zugesetzt sowie 0,3 ml L-Aminosäure-Transaminase-Rohextrakt aus Beispiel 2 - insgesamt 30 µmol/min Enzymaktivität - gegeben. Der Reaktionsansatz wurde mit HCl auf pH 7,4 gestellt und weitere 24 Stunden bei 37°C unter Schütteln inkubiert.

Die α-Aminosäure-Transaminase-Aktivität wurde mit dem Sigma Test-Kit G 0390 bestimmt. Statt α-Ketoglutarat wurden 12 mmol/l Phenylpyruvat-Natriumsalz eingesetzt.

Mit Hilfe analytischer HPLC auf RP18-Säulen konnten nach beendigter Reaktion 33 mMol/l L-Phosphinothricin gemessen werden.

### Beispiel 4

### Spezifität der L-Aminosäure-Transaminase

Die Spezifität der L-Aminosäure-Transaminase von E.coli ATCC 11303 wurde mit Hilfe des Enzym-Assays zur Transaminasemessung nach dem Sigma Test Kit G 0390 durchgeführt. Als Aminogruppendonator wurde Asparaginsäure verwendet.
α-Ketosäure-2-oxoglutarat wurde durch andere α-Ketosäuren ersetzt (s. Tabelle). Die Anfangsreaktionsgeschwindigkeiten in dem Enzymtest wurden miteinander verglichen, wobei die von Phenylpyruvat als 100 % definiert wurde.
Nachfolgende Tabelle zeigt die gemessenen Aktivitäten:

| α-Ketosäure | Transaminaseaktivität (%) |
|---|---|
| Phenylpyruvat | 100 |
| 3-Carboxy-3-oxo-propylmethylphosphinat | 110 |
| 2-Oxoglutarat | 96 |
| Dimethylpyruvat | 82 |
| Phenylglyoxylat | 5 |
| 4-Hydroxy-phenylglyoxylat | 5 |
| Thiophenylglyoxylat | 2 |

### Anhang zu den Beispielen

Gewinnung von D-Aminosäure-Transaminase aus Bacillus licheniformis ATCC 9945 gemäß DOS 34 47 023, Beispiele 1 bis 4:

### Beispiel 1

Der Stamm Bacillus licheniformis ATCC 9945 wird auf Schrägröhrchen mit folgender Zusammensetzung gehalten:
0,3 % Bacto-Beef-Extract
0,5 % Bacto-Peptone
1,5 % Agar
(pH 7,0)
Nach einer Inkubationszeit von 2 - 3 Tagen bei 30°C werden die Sporen mit 10 ml physiologischer Kochsalzlösung abgeschwemmt und 1 ml dieser Suspension zum Animpfen von 100 ml Vorkultur folgender Zusammensetzungen verwendet:
1 % Hefeextrakt
0,8 % Nutrient Broth
0,5 % Maltose
(pH 7,5)
Der Kolben wird bei 190 Upm auf einem Rotationsschüttler 24 Stunden bei 3°C inkubiert. Anschließend werden 50 ml dieser Vorkultur in 2 l fassende Erlenmeyerkolben mit je 500 ml Nährlösung gebracht und als Hauptkultur für 24 Stunden bei 30°C und 190 Upm geschüttelt:
Hauptkulturmedium:
1 % Hefeextrakt
0,8 % Nutrient Broth
0,5 % D,L-Glutaminsäure
(pH 7,2)
Die D-Aminosäure-Transaminase-(DATA)-Aktivität erreicht in der stationären Wachstumsphase ihr Maximum.

### Beispiel 2

Bacillus licheniformis ATCC 9945 wird wie in Beispiel 1 beschrieben in einer Vorkultur (500 ml) angezogen und nach 24 Stunden in einen 12 l-Fermenter mit 9 l des oben angegebenen Hauptkultur-Nährmediums verimpft. Die Fermentationsdauer beträgt bei 30°C, einer Belüftungsrate von 0,15 vvm und 300 Upm 22 - 26 Stunden. Die DATA-Aktivität entspricht der in Beispiel 1.

### Beispiel 3

Der zur DATA-Isolierung notwendige Zellaufschluß erfolgt auf enzymatischem Wege. Dazu werden die in K-Phosphat-Puffer (pH 7,0, 10 mM + 10 µM Pyridoxalphosphat) aufgenommenen Zellen (0,5 g/ml) mit 1 mg Lysozym/ml Zellsuspension versetzt und 10 - 30 Minuten bei 190 UpM und 30°C inkubiert. Nach mikroskopischer Kontrolle wird der Inkubationsansatz zentrifugiert und der Überstand als Rohextrakt weiterverarbeitet. Die Enzymaktivität im Rohextrakt beträgt 0,5 µmol/min·ml.

### Beispiel 4

Der nach Beispiel 3 erhaltene Rohextrakt wird einer fraktionierten Ammonsulfat-Fällung unterworfen:
208 ml des Rohextraktes werden ad 30 % der Sättigung mit Ammonsulfat versetzt und zentrifugiert. Der Überstand wird ad 60 % der Sättigung weiter mit Ammonsulfat versetzt und abzentrifugiert. Der Niederschlag wird in 22 ml Phosphatpuffer (10 mM, pH 8,0), der 10 µM Pyridoxalphosphat enthält, aufgenommen und gegen den gleichen Puffer eine Nacht dialysiert.

## Patentansprüche

1. Verfahren zur Herstellung von L-2-Amino-4-methylphosphinobuttersäure, dadurch gekennzeichnet, daß D,L-2-Amino-4-methyl-phosphinobuttersäure in Gegenwart von D-Aminosäure-Transaminase und L-Aminosäure-Transaminase mit α-Ketosäuren und Aminogruppendonatoren umgesetzt wird.

2. Verfahren nach Anspruch 1, dadurch gekennzeichnet, daß als α-Ketosäure Phenylglyoxylat, 4-Hydroxyphenylglyoxylat und Thiophenylglyoxylat eingesetzt werden.

3. Verfahren nach Anspruch 1 oder 2, dadurch gekennzeichnet, daß als Aminogruppendonator D,L-2-Amino-4-methylphosphinobuttersäure, L-Asparagin, L-Asparaginsäure, L-Glutaminsäure und Glycin eingesetzt werden.

4. Verfahren nach einem oder mehreren der Ansprüche 1 bis 3, dadurch gekennzeichnet, daß die D-Aminosäure-Transaminase aus Bacillus licheniformis ATCC 9945 eingesetzt wird.

5. Verfahren nach einem oder mehreren der Ansprüche 1 bis 4, dadurch gekennzeichnet, daß die L-Aminosäure-Transaminase aus E. coli ATCC 11303 eingesetzt wird.

6. Verfahren nach einem oder mehreren der Ansprüche 1 bis 5, dadurch gekennzeichnet, daß die Reaktion bei einem pH-Wert zwischen 5 und 9 durchgeführt wird.

7. Verfahren nach Anspruch 6, dadurch gekennzeichnet, daß der pH-Wert zwischen 7 und 8,5 liegt.

8. Verfahren nach einem oder mehreren der Ansprüche 1 bis 7, dadurch gekennzeichnet, daß die Reaktionstemperatur zwischen 20 und 65°C liegt.

## Claims

1. A process for the preparation of L-2-amino-4-methylphosphinobutyric acid, which comprises reacting D,L-2-amino-4-methylphosphinobutyric acid with α-keto acids and amino-group donors in the presence of D-aminoacid transaminase and L-aminoacid transaminase.

2. The process as claimed in claim 1, wherein phenylglyoxylate, 4-hydroxyphenylglyoxylate and thiophenylglyoxylate are employed as α-keto acid.

3. The process as claimed in claim 1 or 2, wherein D,L-2-amino-4-methylphosphinobutyric acid, L-asparagine, L-aspartic acid, L-glutamic acid and glycine are employed as amino-group donor.

4. The process as claimed in one or more of claims 1 to 3, wherein the D-aminoacid transaminase from Bacillus licheniformis ATCC 9945 is employed.

5. The process as claimed in one or more of claims 1 to 4, wherein the L-aminoacid transaminase from E. coli ATCC 11303 is employed.

6. The process as claimed in one or more of claims 1 to 5, wherein the reaction is carried out at a pH between 5 and 9.

7. The process as claimed in claim 6, wherein the pH is between 7 and 8.5.

8. The process as claimed in one or more of claims 1 to 7, wherein the reaction temperature is between 20 and 65°C.

## Revendications

1. Procédé pour la préparation de l'acide L-2-amino-4-méthyl-phosphinobutyrique, caractérisé en ce que l'on fait réagir de l'acide D,L-2-amino-4-méthyl-phosphinobutyrique en présence de D-aminoacide transaminase et de L-aminoacide transaminase avec des α-céto-acides et des donneurs de groupe amino.

2. Procédé selon la revendication 1, caractérisé en ce que, comme α-céto-acide, on utilise le phénylglyoxylate, le 4-hydroxyphénylglyoxylate et le thiophénylglyoxylate.

3. Procédé selon la revendication 1 ou 2, caractérisé en ce que, comme donneur de groupe amino, on utilise l'acide D,L-2-amino-4-méthylphosphinobutyrique, la L-asparagine, l'acide L-aspartique, l'acide L-glutamique et la glycine.

4. Procédé selon une ou plusieurs des revendications 1 à 3, caractérisé en ce que l'on utilise la D-aminoacide transaminase provenant de Bacillus licheniformis ATCC 9945.

5. Procédé selon une ou plusieurs des revendications 1 à 4, caractérisé en ce que l'on utilise la L-aminoacide transaminase provenant de E. coli ATCC 11303.

6. Procédé selon une ou plusieurs des revendications 1 à 3, caractérisé en ce que la réaction est effectuée à un pH entre 5 et 9.

7. Procédé selon la revendication 6, caractérisé en ce que le pH se situe entre 7 et 8,5.

8. Procédé selon une ou plusieurs des revendications 1 à 3, caractérisé en ce que la température de la réaction est comprise entre 20 et 65°C.
